(19) Europäisches Patentamt
European Patent Office
Office européen des brevets

(11) Veröffentlichungsnummer: **0 095 719**
**B1**

# (12) EUROPÄISCHE PATENTSCHRIFT

(45) Veröffentlichungstag der Patentschrift: **04.09.85**

(21) Anmeldenummer: **83105162.8**

(22) Anmeldetag: **25.05.83**

(51) Int. Cl.⁴: **C 07 C 49/417**, C 07 C 49/683, C 07 C 131/06, A 61 K 31/12, A 61 K 31/15, B 01 J 19/12

(54) **2-(Z)-(Benzal)-cycloheptan-1-derivate, Verfahren zu ihrer Herstellung, ihre Verwendung, Verfahren zur Herstellung von 2-(Z)-(Benzal)-1-(E)-(aminoalkoxyimino)-cycloheptanen und die ersteren enthaltende Arzneimittel.**

(30) Priorität: **25.05.82 HU 168082**

(43) Veröffentlichungstag der Anmeldung: **07.12.83 Patentblatt 83/49**

(45) Bekanntmachung des Hinweises auf die Patenterteilung: **04.09.85 Patentblatt 85/36**

(84) Benannte Vertragsstaaten: **AT BE CH DE FR GB IT LI NL SE**

(56) Entgegenhaltungen:
**DE - B - 2 609 017**
**SU - A - 633 472**
**US - A - 4 017 637**
**US - A - 4 077 999**

**Chemical Abstracts Band 91, Nr- 21, 19. November 1979, Columbus, Ohio, USA K. ONO et al. "Studies on vasodilators. I. Synthesis and stereochemistry of the metabolites of bencyclane", Seite 622, Spalte 1, Abstract Nr. 174879n**
**Chemical Abstracts Band 87, Nr. 25, 19 December 1977, Columbus, Ohio, USA D.A. LIGHTNER et al. "Synthesis, photooxidation and Z=E photoisomerization of benzalpyrrolinones", Seite 635, Spalte 1, Abstract Nr. 200433b**

(73) Patentinhaber: **EGYT GYOGYSZERVEGYESZETI GYAR, Keresztúri ut 30-38, Budapest X (HU)**

(72) Erfinder: **Budai, Zoltán, Dr., Lukács u. 3, Budapest II (HU)**
Erfinder: **Mezei, Tibor, Dr., Borz u. 4, Budapest XXII (HU)**
Erfinder: **Lay geb. Kónya, Aranka, Dr., Jerney u. 14, Budapest XIV (HU)**

(74) Vertreter: **Beszédes, Stephan G. Dr., Münchener Strasse 80a Postfach 1168, D-8060 Dachau (DE)**

**EP 0 095 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-(Z)-[Benzal]-cycloheptan-1-derivate, ein Verfahren zu ihrer Herstellung, ihre Verwendung, ein Verfahren zur Herstellung von neuen 2-(Z)-[Benzal]-1-(E)-[aminoalkoxyimino]-cycloheptanen und die ersteren enthaltende Arzneimittel, insbesondere solche mit einer Wirkung gegen Depressionen beziehungsweise Antidepressantwirkung, Antiparkinsonwirkung, krampfhemmender Wirkung, lokalanästhetischer Wirkung, antiepileptische Wirkung, die Nicotinsterblichkeit hemmender Wirkung und Tetrabenazin und Reserpin entgegenwirkender Wirkung.

Die Verbindungen 2-[Benzal]-cycloheptan-1-on und 2-[Benzal]-cycloheptan-1-oxim sind in der deutschen Auslegeschrift Nr. 2609017 (entsprechend der ungarischen Patentschrift Nr. 169298) sowie in den US-Patentschriften Nrn. 4017637 und 4077999 und in der sowjetischen Patentschrift Nr. 633472 ohne nähere Angaben als Zwischenprodukte zur Herstellung der basischen Oximäther 2-[Benzal]-1-[aminoalkoxyimino]-cycloheptane erwähnt. Über Isomere dieser Verbindungen ist jedoch nichts angegeben.

Es ist auch bekannt, dass Ketone mit aus einem Hydroxylaminsalz in Gegenwart einer Base *in situ* gebildeten Hydroxylamin reagieren und die entsprechenden Oxime bilden. Je nach der Struktur des als Ausgangsstoff eingesetzten Ketones können im Laufe der Umsetzung mindestens 2 Isomere gebildet werden. Zur Unterscheidung dieser Isomer werden in einfacheren Fällen die Bezeichnungen syn und anti beziehungsweise bei komplizierten Verbindungen die Bezeichnungen Z und E verwendet.

Ferner ist es bekannt, dass verschiedene Isomere unterschiedliche biologische Wirkungen ausüben. Die Herstellung von reinen Isomeren ist deshalb in mehreren Fällen sehr wichtig und dieses Ziel kann nur bei Verwendung von isomerfreien Zwischenprodukten erreicht werden.

Andererseits war es mit dem bekannten Verfahren nach dem Stand der Technik nicht möglich, die Verbindungen 2-(Z)-[Benzal]-cycloheptan-1-on und 2-(Z)-[Benzal]-cycloheptan-1-[(E)-oxim] zu erhalten. Es war auch nicht möglich, nach der ungarischen Patentanschrift Nr. 169298 die (Z),(E)-Isomere der 2-[Benzal]-[aminoalkoxyimino]-cycloheptane derselben herzustellen, wobei dazu auch kein Weg durch ein Isomerisieren oder eine Trennung führen könnte.

Nach dem genannten Oximherstellungsverfahren wurde von der Anmelderin das 2-[Benzal]-cycloheptan-1-oxim hergestellt und dabei festgestellt, dass das erhaltene Produkt das E,E-Isomer ist.

Weiterhin ist aus Chemical Abstracts, Band 87, Nr. 25, 19. Dezember 1977, Seite 635, Spalte 1, Abstract Nr. 200433b die Synthese, Photooxidation und Z ⇌ E-Photoisomerisation von Benzalpyrrolinonen bekannt. Es handelt sich dabei um eine zum Gleichgewicht führende Reaktion von heterocyclischen Verbindungen.

Ausserdem sind aus Chemical Abstracts, Band 91, Nr. 21, 19. November 1979, Seite 622, Spalte 1, Abstract Nr. 174879n 9 Diastereoisomere von Benzcyclan, das heisst von 3-{[1'-(Benzyl)-cycloheptyl]-oxy}-N,N-dimethylpropylamine mit einer Hydroxy- oder Carbonylgruppe an dessen Cycloheptanring als Vasodilatatoren bekannt.

Der Erfindung liegt daher die Aufgabe zugrunde, überlegene pharmakologische Wirkungen aufweisende neue Isomere von 2-[Benzal]-cycloheptan-1-on und 2-[Benzal]-cycloheptan-1-oxim, ein einfaches chemisch eigenartiges Verfahren zur Herstellung derselben in hoher Reinheit und guter Ausbeute, ihre Verwendung, ein Verfahren zur Herstellung von neuen 2-(Z)-[Benzal]-1-(E)-[aminoalkoxyimino]-cycloheptanen über die ersteren und die ersteren enthaltenden Arzneimittel zu schaffen.

Das Obige wurde überraschenderweise durch die Erfindung erreicht.

Es wurde nämlich überraschenderweise festgestellt, dass das 2-(E)-[Benzal]-cycloheptan-1-on in Lösung in einem inerten Lösungsmittel beim Bestrahlen mit Licht mit einer Wellenlänge von 200 bis 600 nm als Folge der Photoisomerisation in das 2-(Z)-[Benzal]-cycloheptan-1-on überführt wird, welches aus dem inerten Lösungsmittel gewonnen und/oder, gegebenenfalls ohne Isolieren, in das 2-(Z)-[Benzal]-cycloheptan-1-[(E)-oxim] überführt werden kann.

Gegenstand der Erfindung sind daher 2-(Z)-[Benzal]-cycloheptan-1-derivate der allgemeinen Formel

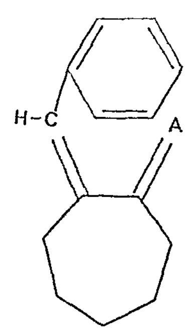

(I)

worin:

A für ein Sauerstoffatom oder eine Hydroxyiminogruppe steht.

Anders ausgedrückt handelt es sich dabei um die Verbindungen 2-(Z)-[Benzal]-cycloheptan-1-on und 2-(Z)-[Benzal]-cycloheptan-1-[(E)-oxim].

Gegenstand der Erfindung ist auch ein Verfahren zur Herstellung der erfindungsgemässen 2-(Z)-[Benzal]-cycloheptan-1-derivate, welches dadurch gekennzeichnet ist, dass 2-(E)-[Benzal]-cycloheptan-1-on der Formel:

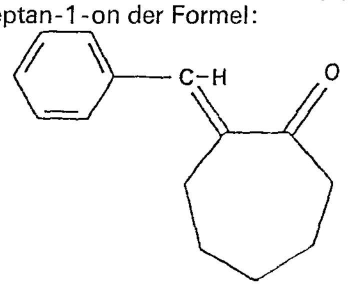

(II)

in einem inerten Lösungsmittel einem Photoisomersieren durch Bestrahlen mit Licht mit einer Wellenlänge von 200 bis 600 nm unterworfen wird und danach das erhaltene 2-(Z)-[Benzal]-cycloheptan-1-on (Formel I mit A = Sauerstoffatom) gewonnen und/oder in an sich bekannter Weise in das entsprechende Oxim 2-(Z)-[Benzal]-cycloheptan-1-[(E)-oxim] (Formel I mit A = Hydroxyiminogruppe) überführt wird.

Gegenüber Chemical Abstracts, Band 87, Nr. 25, 19. Dezember 1977, Seite 635, Spalte 1, Abstract Nr. 200433b, wonach mit den dort beschriebenen Verbindungen nur eine zum Gleichgewicht führende Reaktion stattfindet, ist es überraschend, dass im erfindungsgemässen Verfahren bei der als Ausgangssubstanz verwendeten cycloaliphatischen Verbindung 2-(E)-[Benzal]-cycloheptan-1-on der Formel II die E → Z-Umwandlung nur in einer Richtung, nämlich zum erfindungsgemässen Z-Isomer 2-(Z)-[Benzal]-cycloheptan-1-on der Formel I verläuft. Generell wurde der Durchschnittsfachmann dadurch von der Durchführung der erfindungsgemässen Photoisomerisation abgehalten, dass die Photoisomerisation von Verbindungen mit ähnlicher Struktur als die der im erfindungsgemässen Verfahren als Ausgangssubstanzen verwendeten 2-(E)-[Benzal]-cycloheptan-1-one der Formel II nur in ausserordentlich verdünnten (etwa 0,8 gew.-%igen) Lösungen durchgeführt werden konnte und sie nur zu einem Gleichgewicht führte, indem im jeweiligen Produkt insgesamt 30 Gew.-% Ausgangsisomer E verblieben, dessen Menge nur in der Weise auf 5 Gew.-% gesenkt werden konnte, dass die Photoisomerisation in einer noch verdünnteren (etwa 0,07 gew.-%igen) Lösung durchgeführt wurde (vergleiche J.C.S. Perkin I [1975], Seiten 102 bis 113). Für eine präparative Herstellung waren daher diese Verfahren ungeeignet. Im Gegensatz dazu wird im erfindungsgemässen Verfahren das Z-Isomer 2-(Z)-[Benzal]-cycloheptan-1-on der Formel I überraschenderweise aus dem entsprechenden E-Isomer 2-(E)-[Benzal]-cycloheptan-1-on der Formel II in der sehr hohen Ausbeute von 97,5% und in hoher Isomerreinheit erhalten, wobei das genannte E-Isomer in viel höheren Konzentrationen von beispielsweise 20 Gew.-% (ist eine etwa 300mal so hohe Konzentration wie die nach dem Stand der Technik verwendete) verwendet werden können.

Die erfindungsgemässen 2-(Z)-[Benzal]-cycloheptan-1-derivate der allgemeinen Formel I unterscheiden sich von den Verbindungen von Chemical Abstracts, Band 91, Nr. 21, 19. November 1979, Seite 622, Spalte 1, Abstract Nr. 174879n schon darin, dass sie im Gegensatz zu der Benzylgruppe der letzteren eine Benzalgruppe aufweisen. Überdies haben die erfindungsgemässen 2-(Z)-[Benzal]-cycloheptan-1-derivate der allgemeinen Formel I entweder ein Sauerstoffatom oder eine Hydroxyiminogruppe, während die Verbindungen der genannten Druckschrift, sofern sie eine Carbonylgruppe (Ketogruppe) aufweisen, am Cycloheptanring zusätzlich eine Aminooxygruppe haben. Weiterhin ist in den erfindungsgemässen 2-(Z)-[Benzal]-cycloheptan-1-derivaten, soweit sie eine Ketogruppe haben, diese der Benzalgruppe benachbart, während in den Verbindungen der genannten Druckschrift die Ketogruppe, sofern sie vorhanden ist, in der γ-Stellung zur Benzylgruppe, die ohnehin nicht mit der Benzalgruppe vergleichbar ist, auch was die sterische Lage betrifft, sich befindet. Schliesslich ist festzustellen, dass es sich bei den erfindungsgemässen 2-(Z)-[Benzal]-cycloheptan-1-derivaten der allgemeinen Formel I um (Z)-Isomere handelt, während bei den Verbindungen der genannten Druckschrift (Z)- und (E)-Isomere nicht möglich sind, da sie keine C = C − Doppelbindung aufweisen.

Vorzugsweise wird das Bestrahlen mit Licht mit einer Wellenlänge von 300 bis 600 nm durchgeführt.

Es ist auch bevorzugt, als inertes Lösungsmittel einen aliphatischen Alkohol, insbesondere Methanol und/oder Äthanol, zu verwenden.

Das gegebenenfalls erfolgende Überführen des 2-(Z)-[Benzal]-cycloheptan-1-ones in das Oxim 2-(Z)-[Benzal]-cycloheptan-1-[(E)-oxim] kann mit aus einem Hydroxylaminsalz, vorzugsweise Hydroxylaminsulfat oder -hydrochlorid, *in situ* gebildetem Hydroxylamin in einem wässerigen und/oder alkoholischen Medium durchgeführt werden. Zu diesem Zweck kann beziehungsweise können vorteilhaft 1 oder mehr geradkettige[r] oder verzweigte[r], mit Wasser unbegrenzt mischbare[r] aliphatische[r] Alkohol(e), zum Beispiel Methanol, Äthanol, n-Propanol und/oder Isopropanol, insbesondere Äthanol, eingesetzt werden.

Die genannte Umsetzung wird zweckmässig in Gegenwart von Basen durchgeführt. Zu diesem Zweck können organische Basen, beispielsweise Pyridin und/oder 1 oder mehr Pyridinbase(n), das heisst substituierte[s] Pyridin(e), wie 2-, 2- und/oder 4-Picolin(e) und/oder 1 oder mehr Lutidin(e), oder 1 oder mehr anorganische Base(n), beispielsweise Alkalimetallhydroxyd(e), Alkalimetallcarbonat(e) und/oder Alkalimetallbicarbonat(e), verwendet werden.

Die Reaktionstemperatur beeinflusst die Ausbeute nicht wesentlich und kann innerhalb weiter Grenzen variiert werden. Vorteilhaft wird die genannte Umsetzung des erfindungsgemässen Verfahrens bei Temperaturen von 20 bis 90°C, insbesondere 75 bis 85°C, durchgeführt.

Wenn ein besonders reines Produkt erwünscht ist, kann das bei der Reaktion erhaltene Rohprodukt nach an sich bekannten Verfahrensweisen, zum Beispiel durch Säulenchromatographie, Destillation, Zonenschmelzen und/oder Kristallisieren, beispielsweise aus Petroläther oder Äthanol, gereinigt werden.

Das im erfindungsgemässen Verfahren als Ausgangssubstanz verwendete 2-(E)-[Benzal]-cycloheptan-1-on der Formel II ist bekannt und kann nach im Schrifttum beschriebenen Verfahren (zum Beispiel Gazz. Chim. Ital. *91* [1961], 326 bis 348) hergestellt worden sein. Die Hydroxylaminsalze, wie Hydroxylaminsulfat und -hydrochlorid, sind Handelsprodukte.

Die erfindungsgemässen 2-(Z)-[Benzal]-cycloheptan-1-derivate der allgemeinen Formel I sind wertvolle Zwischenprodukte zur Herstellung der ebenfalls pharmakologisch wirksamen, insbesondere eine Wirkung gegen Depressionen beziehungsweise Antidepressantwirkung, Antiparkinsonwirkung, krampfhemmende Wirkung, lokalanästhetische Wirkung, antiepileptische Wirkung, die Nicotinsterblichkeit hemmende Wirkung und Tetrabenazin und Reserpin entgegenwirkende Wirkung aufweisenden, neuen basischen Oximäther 2-(Z)-[Benzal]-1-(E)-[aminoalkoxyimino]-cycloheptane der allgemeinen Formel:

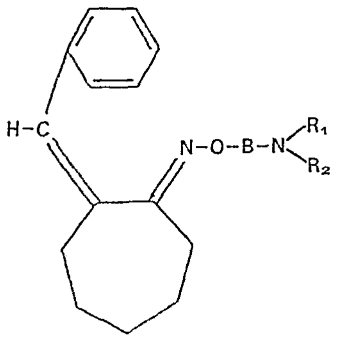

(III)

worin:

B für einen geradkettigen oder verzweigten Alkylenrest mit 2 bis 4 Kohlenstoffatomen steht, und

$R_1$ und $R_2$ Wasserstoff oder einen Alkylrest mit 1 bis 4 Kohlenstoffatom(en) bedeuten, oder

$R_1$ und $R_2$ zusammen eine, gegebenenfalls durch ein Sauerstoffatom oder ein, gegebenenfalls durch einen Alkylrest mit 1 bis 3 Kohlenstoffatom(en) oder einen Benzylrest substituiertes, Stickstoffatom unterbrochene, Alkylenkette mit 4 bis 7 Kohlenstoffatomen darstellen.

Gegenstand der Erfindung ist daher auch die Verwendung der erfindungsgemässen 2-(Z)-[Benzal]-cycloheptan-1-derivate zur Herstellung von 2-(Z)-[Benzal]-1-(E)-[aminoalkoxyimino]-cycloheptanen der allgemeinen Formel:

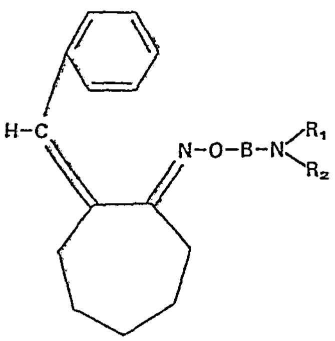

(III)

worin:

B für einen geradkettigen oder verzweigten Alkylenrest mit 2 bis 4 Kohlenstoffatomen steht, und

$R_1$ und $R_2$ Wasserstoff oder einen Alkylrest mit 1 bis 4 Kohlenstoffatom(en) bedeuten, oder

$R_1$ und $R_2$ zusammen eine, gegebenenfalls durch ein Sauerstoffatom oder ein, gegebenenfalls durch einen Alkylrest mit 1 bis 3 Kohlenstoffatom(en) oder einen Benzylrest substituiertes, Stickstoffatom unterbrochene, Alkylenkette mit 4 bis 7 Kohlenstoffatomen darstellen,

a) durch in an sich bekannter Weise erfolgendes Umsetzen des 2-(Z)-[Benzal]-cycloheptan-1-ones (Formel I mit A = Sauerstoffatom) mit O-(Aminoalkyl)-hydroxylaminderivaten der allgemeinen Formel:

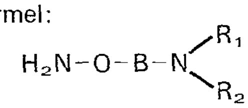

(IV)

worin B, $R_1$ und $R_2$ wie oben festgelegt sind, oder

b) durch in an sich bekannte Weise erfolgendes Umsetzen des 2-(Z)-[Benzal]-cycloheptan-1-[(E)-oxim]-es (Formel I mit A = Hydroxyiminogruppe),

α) mit Halogenalkylaminen der allgemeinen Formel:

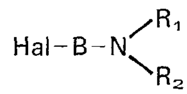

(V)

worin:

Hal für ein Halogenatom, insbesondere Chloratom, steht und

B, $R_1$ und $R_2$ wie oben festgelegt sind, oder

β) durch Umsetzen des ersteren mit einem Dihalogenalkan der allgemeinen Formel:

Hal–B–Hal' (VI)

worin:

Hal und Hal' für gleiche oder verschiedene Halogenatome, insbesondere Chloratome, stehen und

B wie oben festgelegt ist,

und Aminieren der erhaltenen Halogenalkyläther.

Ferner ist Gegenstand der Erfindung ein Verfahren zur Herstellung der 2-(Z)-[Benzal]-1-(E)-[aminoalkoxyimino]-cycloheptane der allgemeinen Formel III, welches dadurch gekennzeichnet ist, dass 2-(E)-[Benzal]-cycloheptan-1-on der Formel:

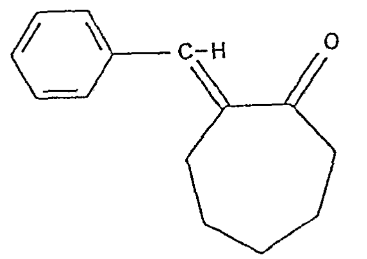

(II)

in einem inerten Lösungsmittel einem Photoisomerisieren durch Bestrahlen mit Licht mit einer Wellenlänge von 200 bis 600 nm unterworfen wird und danach das erhaltene, gegebenenfalls gewonnene, 2-(Z)-[Benzal]-cycloheptan-1-on (Formel I mit A = Sauerstoffatom),

a) in an sich bekannte Weise mit O-(Aminoalkyl)-hydroxylaminderivaten der allgemeinen Formel:

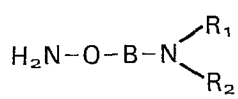

(IV)

worin B, $R_1$ und $R_2$ wie oben festgelegt sind, umgesetzt wird, oder

b) in das entsprechende Oxim 2-(Z)-[Benzal]-cycloheptan-1-[(E)-oxim] (Formel I mit A = Hydroxyiminogruppe) überführt wird und dieses:

α) mit Halogenalkylaminen der allgemeinen Formel:

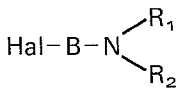 (V)

worin:

Hal für ein Halogenatom, insbesondere Chloratom, steht, und

B, $R_1$ und $R_2$ wie oben festgelegt sind,
umgesetzt wird oder

β) mit Dihalogenalkanen der allgemeinen Formel:

$$Hal-B-Hal' \quad (VI)$$

worin:

Hal und Hal' für gleiche oder verschiedene Halogenatome, insbesondere Chloratome, stehen und

B wie oben festgelegt ist,
umgesetzt wird und die erhaltenen Halogenalkyläther aminiert werden.

Im Gegensatz zu den Verbindungen der deutschen Auslegeschrift Nr. 2609017, welche nicht die Z-E-Isomere sind, sind die 2-(Z)-[Benzal]-1-1(E)-[aminoalkoxyimino]-cycloheptane der allgemeinen Formel III, zu deren Herstellung die erfindungsgemässen 2-(Z)-[Benzal]-cycloheptan-1-derivate der allgemeinen Formel I verwendet werden können, Z,E-Isomere.

Derselbe Unterschied gilt auch gegenüber den US-Patentschriften Nrn. 4017637 und 4077999. Ein weiterer Unterschied gegenüber der US-Patentschrift Nr. 4017637 besteht darin, dass die Verbindungen der letztgenannten Druckschrift Aminobenzylcycloalkanole sind, während die 2-(Z)-[Benzal]-1-(E)-[aminoalkoxyimino]-cycloheptane der allgemeinen Formel III Benzalaminoalkoxyiminocycloalkane sind. Dies bedeutet, dass die 2-(Z)-[Benzal]-1-(E)-[aminoalkoxyimino]-cycloheptane der allgemeinen Formel III im Gegensatz zu den Verbindungen der US-Patentschrift Nr. 4017637, welche eine Benzylgruppe, die am Kohlenstoffatom ihres Methylenteiles zwingend durch eine Aminogruppe substituiert ist, aufweisen, eine Benzalgruppe haben. Ferner handelt es sich bei den 2-(Z)-[Benzal]-1-(E)-[aminoalkoxyimino]-cycloheptane der allgemeinen Formel III im Gegensatz zu den Verbindungen der US-Patentschrift Nr. 4017637, welche Alkohole, Äther mit aliphatischen Alkoholen oder Ester mit aliphatischen Carbonsäuren sind, um Oximinoverbindungen. Auch wäre in gewissem Gegensatz zur in Spalte 6, Zeile 57 bis Spalte 7, Zeile 2 der US-Patentschrift Nr. 4017637 beschriebenen Trennung der cis- und trans-Isomere der dort beschriebenen Verbindungen, deren Isomerie ohnehin nicht mit der der (Z)- und (E)-Isomere im vorliegenden Fall vergleichbar ist, da es sich in der genannten Druckschrift um die relativen sterischen Stellungen des Restes $OR_1$ und der Wasserstoffatome $H_A$ und $H_B$ und nicht um die relativen sterischen Stellungen einer Benzalgruppe und einer Aminoalkoxyiminogruppe oder einer damit vergleichbaren Gruppe handelt, keine Trennung der den 2-(Z)-[Benzal]-1-(E)-[aminoalkoxyimino]-cycloheptanen der allgemeinen Formel III entsprechenden (Z)- und (E)-Isomerengemische möglich.

Von den in Chemical Abstracts, Band 91, Nr. 21, 19. November 1979, Seite 622, Spalte 1, Abstract Nr. 174879n unterscheiden sich die 2-(Z)-[Benzal]-1-(E)-[aminoalkoxyimino]-cycloheptane der allgemeine Formel III, zu deren Herstellung die erfindungsgemässen 2-(Z)-[Benzal]-cyycloheptan-1-derivate der allgemeinen Formel I verwendet werden können, zunächst darin, dass die 2-(Z)-[Benzal]-1-(E)-[aminoalkoxyimino]-cycloheptane der allgemeinen Formel III im Gegensatz zu den Verbindungen der genannten Druckschrift, welche eine Benzylgruppe haben, eine Benzalgruppe aufweisen. Auch haben die 2-(Z)-[Benzal]-1-(E)-[aminoalkoxyimino]-cycloheptane der allgemeinen Formel III eine Aminoalkoxyiminogruppe, während die Verbindungen der genannten Druckschrift keine solche aufweisen, sondern nur eine Aminooxygruppe. Schliesslich haben die 2-(Z)-[Benzal]-1-(E)-[aminoalkoxyimino]-cycloheptane der allgemeinen Formel III weder eine Hydroxygruppe noch eine Carbonylgruppe, während in den Verbindungen der genannten Druckschrift 1 derselben zwingend vorliegen muss.

Zweckmässig wird die Umsetzung der erfindungsgemässen 2-(Z)-[Benzal]-cycloheptan-1-derivate der allgemeinen Formel I mit den Halogenalkylaminen der allgemeinen Formel V beziehungsweise Dihalogenalkanen der allgemeinen Formel VI in inerten Lösungsmitteln in Gegenwart von basischen Kondensationsmitteln durchgeführt. Als inerte Lösungsmittel können aromatische Kohlenwasserstoffe, wie Benzol, Toluol, Xylole und/oder Cumol, verwendet werden. Als Kondensationsmittel können Alkalimetallamide. wie Natriumamid, oder Alkalimetallhydride, wie Natriumhydrid, verwendet werden. Als andere Möglichkeit können als Kondensationsmittel Alkalimetalle, wie Natrium, verwendet werden. In diesem Falle ist die Verwendung von Alkoholen, wie Äthylalkohol, Propylalkoholen und/oder Butylalkoholen, als Lösungsmitteln bevorzugt. Als Kondensationsmittel können auch Alkalimetallhydroxyde, wie Natriumhydroxyd, verwendet werden, in welchem Falle als Kondensationsmittel auch Wasser verwendbar ist.

Beispielsweise können die erfindungsgemässen 2-(Z)-[Benzal]-cycloheptan-1-derivate der allgemeinen Formel I so in 2-(Z)-[Benzal]-1-(E)-[2'-{dimethylamino}-äthoxyimino]-cycloheptan, 2-(Z)-[Benzal]-1-(E)-[3'-{dimethylamino}-propoxyimino]-cycloheptan, 2-(Z)-[Benzal]-1-(E)-[2'-{diisopropylamino}-äthoxyimino]-cycloheptan und 2-(Z)-[Benzal]-1-(E)-[3'-{4''-⟨methyl⟩-piperazinyl}-propoxyimino]-cycloheptan als 2-(Z)-[Benzal-1-(E)-[aminoalkoxyimino]-cycloheptane überführt werden.

Ferner sind erfindungsgemäss Arzneimittel, welche eine oder beide der erfindungsgemässen Verbindungen als Wirkstoff(e), gegebenenfalls zusammen mit einem oder üblichen Konfektionierungsmittel(n), enthalten, vorgesehen.

Diese Verbindungen haben nämlich wie bereits erwähnt wertvolle pharmakologische Wirkungen, insbesondere eine Wirkung gegen Depressionen beziehungsweise Antidepressantwirkung, Antiparkinsonwirkung, krampfhemmende Wirkung, lokalanästhetische Wirkung, die Nicotinsterblichkeit hemmende Wirkung und Tetrabenazin und Reserpin entgegenwirkende Wirkung. Dabei hat das erfindungsgemässe 2-(Z)-[Benzal]-cycloheptan-1-on (Formel I mit A = Sauerstoffatom) vor allem eine lokalanästhetische und antiepileptische Wirkung und eine Wirkung gegen Depressionen beziehungsweise Antidepressantwirkung.

Die Erfindung wird anhand der folgenden Beispiele näher erläutert, wobei die Beispiele 1 bis 3 die Herstellung von erfindungsgemässen 2-(Z)-[Benzal]-cycloheptan-1-derivaten und die Beispiele 4 bis 7 die Verwendung des einen derselben zur Herstellung von 2-(Z)-[Benzal]-1-(E)-[aminoalkoxyimino]-cycloheptanen betreffen.

*Beispiel 1 :*

*2-(Z)-[Benzal]-cycloheptan-1-on*

Es wurde eine Lösung von 40 g (0,2 Mol) 2-(E)-[Benzal]-cycloheptan-1-on in 400 ml Methanol unter kräftigem Rühren beziehungsweise Schütteln mit einer Quecksilberdampflampe (150 W) bis zur Vervollständigung der Überführung beleuchtet (die Reaktionsvorrichtung bestand aus Glas vom Typ „Duran 50"). Danach wurde die Lösung geklärt, filtriert und eingedampft. So wurden 39 g 97,5% der Theorie 2-(Z)-[Benzal]-cycloheptan-1-on mit einem $[n]_D^{20}$-Wert von 1,5692 erhalten.

*Analyse* für $C_{14}H_{16}O$ (Molekulargewicht = 200,28):

| | | |
|---|---|---|
| Berechnet: | C 83,94 | H 8,06% |
| Gefunden: | C 83,51 | H 7,95% |

UV-Spektrum: $\lambda_{max}$ = 276 nm ($\varepsilon$ = 8240).
Magnetisches Kernresonanzspektrum [NMR] ($CDCl_3$): 6,9 *s* (5H); 2,3 *b* (2H); 6,12 *s* (H); 1,6 *b* (6H); 2,5 *b* (2H).

*Beispiel 2:*

*2-(Z)-[Benzal]-cycloheptan-1-[(E)-oxim]*

Es wurden 5 g (0,025 Mol) wie im Beispiel 1 beschrieben hergestelltes 2-(Z)-[Benzal]-cycloheptan-1-on in 25 ml Äthanol gelöst. Zur Lösung wurden 1,91 g (0,025 Mol) Hydroxylaminhydrochlorid und danach unter kräftigem Rühren beziehungsweise Schütteln 1,49 g (0,014 Mol) feingepulvertes Natriumcarbonat zugegeben. Das Reaktionsgemisch wurde zum Sieden erhitzt und bis zum Ende der Kohlendioxydentwicklung aus dieser Temperatur gehalten. Das Gemisch wurde auf 100 g zerstossenes Eis gegossen und die ausgeschiedenen Kristalle wurden abfiltriert. So wurden 5,04 g (93,6% der Theorie) 2-(Z)-[Benzal]-cycloheptan-1-[(E)-oxim] mit einem Schmelzpunkt von 114 bis 116°C erhalten.

*Analyse* für $C_{14}H_{17}NO$ (Molekulargewicht = 215,29):

| | | | |
|---|---|---|---|
| Berechnet: | C 78,10 | H 7,96 | N 6,50% |
| Gefunden: | C 77,83 | H 7,79 | N 6,52% |

UV-Spektrum: $\lambda_{max}$ = 255 nm ($\varepsilon$ = 13 312).
Magnetisches Kernresonanzspektrum [NMR] ($CDCl_3$): 6,86 *s* (5H); 2,25 *bs* (2H); 6,12 *s* (H); 1,52 *bs* (6H); 2,5 *bs* (2H).

*Beispiel 3:*

*2-(Z)-[Benzal]-cycloheptan-1-[(E)-oxim]*

Es wurden zu einer Lösung von 5 g (0,025 Mol) wie im Beispiel 1 beschrieben hergestelltem 2-(Z)-[Benzal]-cycloheptan-1-on in 25 ml Methanol zunächst 1,91 g (0,0275 Mol) Hydroxylaminhydrochlorid und danach 2,18 g (0,0275 Mol) Pyridin zugegeben. Das Reaktionsgemisch wurde einige Stunden lang bei Raumtemperatur gerührt beziehungsweise geschüttelt und dann auf 35 g zerstossenes Eis gegossen. So wurden 4,55 g (84,5% der Theorie) 2-(Z)-[Benzal]-cycloheptan-1-[(E)-oxim] in Form von weissen Kristallen erhalten. Die Daten dieses Produktes waren mit denen der nach dem Beispiel 2 hergestellten Verbindung identisch.

*Beispiel 4:*

*2-(Z)-[Benzal]-1-(E)-[2'-{-dimethylamino}'-äthoxyimino]-cycloheptan*

Es wurde einer Suspension von 2,4 g (0,1 Mol) Natriumhydrid in 50 ml absolutem Toluol bei 85°C unter ständigem Rühren beziehungsweise Schütteln eine Lösung von 21,5 g (0,1 Mol) 2-(Z)-[Benzal]-cycloheptan-1-on in 200 ml absolutem Toluol zugetropft. Dann wurde das Gemisch 2 Stunden lang auf 130°C gehalten, worauf unter Weiterrühren beziehungsweise -schütteln eine 40 gew.-%ige Lösung von 11,8 g (0,11 Mol) 2-(Dimethylamino)-äth-1-ylchlorid in absolutem Toluol zugesetzt wurde. Nach noch 6 Stunden langem Erhitzen wurde die toluolische Lösung auf 30°C gekühlt und mit 100 ml Wasser gewaschen, worauf mit einer wässerigen Lösung von 15 g (0,1 Mol) Weinsäure oder mit einer wässerigen verdünnten Salzsäure in einer 0,11 Mol entsprechenden Menge extrahiert wurde. Danach wurde die auf 0 bis 5°C gekühlte wässerige Lösung mit konzentriertem Ammoniumhydroxyd auf einem pH-Wert von 10 alkalisch gemacht. Die in Form eines Öles ausgeschiedene Base wurde mit Dichloräthan extrahiert und das Lösungsmittel wurde abgedampft.

So wurden 20 g (69,6% der Theorie) 2-(Z)-[Benzal]-1-(E)-[2'-{dimethylamino}-äthoxyimino]-cycloheptan erhalten.

Aus diesem konnte das entsprechende Fumarat 2-(Z)-[Benzal]-1-(E)-[2'-{dimethylamino}-äthoxyimino]-cycloheptanfumarat wie folgt hergestellt werden.

Es wurden 19,7 g (0,068 Mol) 2-(Z-[Benzal]-1-(E)-[2'-{dimethylamino}-äthoxyimino]-cycloheptanbase in 20 ml kaltem Aceton gelöst und zur Lösung wurde eine Lösung von 7,9 g (0,068 Mol) Fumarsäure in 80 ml heissem absolutem Alkohol zugegeben. Das beim Abkühlen ausgeschieden

weisse kristalline 2-(Z)-[Benzal]-1-(E)-[2'-{dimethylamino}-äthoxyimino]-cycloheptanfumarat wurde abfiltriert und getrocknet. Es hatte einen Schmelzpunkt von 158 bis 161°C und sein UV-Spektrum hatte einen Wert $\lambda_{max}$ = 255 nm.

*Analyse* für $C_{22}H_{30}N_2O_5$:

| | | | |
|---|---|---|---|
| Berechnet: | C 65,60 | H 7,52 | N 6,98% |
| Gefunden: | C 65,60 | H 7,73 | N 6,87% |

*Beispiel 5:*

*2-(Z)-[Benzal]-1-(E)-[3'-{dimethylamino}-propoxyimino]-cycloheptan*

Beispiel 4 wurde mit dem Unterschied wiederholt, dass an Stelle des 2-(Dimethylamino)-äth-1-ylchorides 13,3 g (0,11 Mol) 3-(Dimethylamino)-n-prop-1-ylchlorid verwendet wurde.

So wurden 16,7 g (72,4% der Theorie) 2-(Z)-[Benzal]-1-(E)-[3'-{dimethylamino}-propoxyimino]-cycloheptan erhalten.

Dieses konnte analog wie im Beispiel 4 beschrieben in das entsprechende Fumarat 2-(Z)-[Benzal]-1-(E)-]3'-{-dimthylamino}-propoxyimino]-cycloheptanfumarat überführt werden.

*Analyse* für $C_{23}H_{32}N_2O_5$:

| | | | |
|---|---|---|---|
| Berechnet: | C 66,34 | H 7,74 | N 6,72% |
| Gefunden: | C 66,23 | H 7,80 | N 6,66% |

*Beispiel 6:*

*2-(Z)-[Benzal]-1-(E)-[2'-{diisopropylamino}-äthoxyimino]-cycloheptan*

Beispiel 4 wurde mit dem Unterschied wiederholt, dass an Stelle des 2-(Dimethylamino)-äth-1-ylchlorides 17,95 g (0,11 Mol) 2-(Diisopropylamino)-äth-1-ylchorid verwendet wurden.

So wurden 26,0 g (76,2% der Theorie) 2-(Z)-[Benzal]-1-(E)-[2'-{diisopropylamino{-[äthoxyimino]-cycloheptan erhalten.

Dieses konnte in der im Beispiel 4 beschriebenen Weise in das entsprechende Fumarat 2-(Z)-[Benzal]-1-(E)-[2'-{diisopropylamino}-äthoxyimino]-cycloheptanfumarat mit einem Schmelzpunkt von 118 bis 120°C und einem UV-Spektrum mit einem Wert $\lambda_{max}$ = 258 nm überführt werden.

*Analyse* für $C_{26}H_{38}N_2O_5$:

| | | | |
|---|---|---|---|
| Berechnet: | C 68,08 | H 8,35 | N 6,11% |
| Gefunden: | C 68,16 | H 8,46 | N 6,07% |

*Beispiel 7:*

*2-(Z)-[Benzal]-1-(E)-[3'-{4''-⟨methyl⟩-piperazin-1''-yl}-n-prop-1'-oxyimino]-cycloheptan*

Beispiel 4 wurde mit dem Unterschied wiederholt, dass an Stelle das 2-(Dimethylamino)-äth-1-ylchlorides 19,5 g 0,11 Mol) [3-{4'-⟨methyl⟩-piperazin-1'-yl}-n-prop-1-yl]-chlorid verwendet wurden.

So wurden 26,5 g (72,5% der Theorie) 2-(Z)-[Benzal]-1-(E)-[3'-{4''-⟨methyl⟩-piperazin-1''-yl}-n-prop-1'-oxyimino]-cycloheptan erhalten.

Diese 2-(Z)-[Benzal]-1-(E)-[3'-{4''-⟨methyl⟩-piperazin-1''-yl}-n-prop-1'-oxyimino]-cycloheptanbase konnte analog wie im Beispiel 4 beschrieben in das entsprechende Difumarat 2-(Z)-[Benzal]-1-(E)-[3'-{4''-⟨methyl⟩-piperazin-1''-yl}-n-prop-1'-oxyimino]-cycloheptandifumarat überführt werden.

*Analyse* für $C_{30}H_{41}N_3O_9$:

| | | | |
|---|---|---|---|
| Berechnet: | C 61,31 | H 7,03 | N 7,15% |
| Gefunden: | C 61,20 | H 6,94 | N 7,10% |

**Patentansprüche** für die Vertragsstaaten BE, CH, DE, FR, GB, IT, LI, NL, SE

1. 2-(Z)-[Benzal]-cycloheptan-1-derivate der allgemeinen Formel:

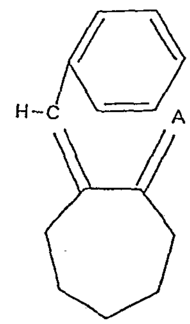

(I)

worin:

A für ein Sauerstoffatom oder eine Hydroxyiminogruppe steht.

2. Verfahren zur Herstellung de 2-(Z)-[Benzal]-cycloheptan-1-derivate nach Anspruch 1, dadurch gekennzeichnet, dass man 2-(E)-[Benzal]-cycloheptan-1-on der Formel:

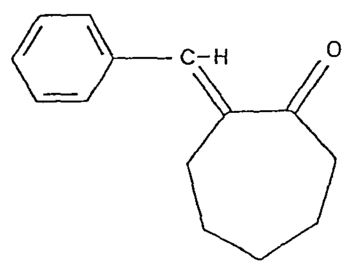

(II)

in einem inerten Lösungsmittel einem Photoisomerisieren durch Bestrahlen mit Licht mit einer Wellenlänge von 200 bis 600 nm unterwirft und danach das erhaltene 2-(Z)-]Benzal]-cycloheptan-1-on (Formel I mit A = Sauerstoffatom) gewinnt und/oder in an sich bekannter Weise in das entsprechende Oxim 2-(Z)-[Benzal]-cycloheptan-1-[(E)-oxim] (Formel I mit A = Hydroxyiminogruppe) überführt.

3. Verfahren nach Anspruch 2, dadurch gekennzeichnet, dass man das Bestrahlen mit Licht mit einer Wellenlänge von 300 bis 600 nm durchführt.

4. Verfahren nach Anspruch 3, dadurch gekennzeichnet, dass man als inertes Lösungsmittel einen aliphatischen Alkohol, insbesondere Methanol, verwendet.

5. Verwendung der 2-(Z)-[Benzal]-cycloheptan-1-derivate nach Anspruch 1 zur Herstellung von 2-(Z)-[Benzal]-1-(E)-[aminoalkoxyimino]-cycloheptanen der allgemeinen Formel:

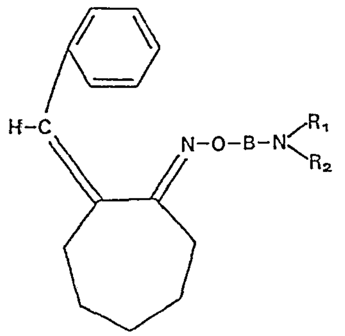 (III)

worin:

B für einen geradkettigen oder verzweigten Alkylenrest mit 2 bis 4 Kohlenstoffatomen steht, und

$R_1$ und $R_2$ Wasserstoff oder einen Alkylrest mit 1 bis 4 Kohlenstoffatom(en) bedeuten, oder

$R_1$ und $R_2$ zusammen eine, gegebenenfalls durch ein Sauerstoffatom oder ein, gegebenenfalls durch einen Alkylrest mit 1 bis 3 Kohlenstoffatom(en) oder einen Benzylrest substituiertes, Stickstoffatom unterbrochene, Alkylenkette mit 4 bis 7 Kohlenstoffatomen darstellen,

a) durch in an sich bekannter Weise erfolgendes Umsetzen des 2-(Z)-[Benzal]-cycloheptan-1-ones (Formel I mit A = Sauerstoffatom) mit O-(Aminoalkyl)-hydroxylaminderivaten der allgemeinen Formel:

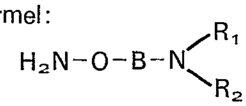 (IV)

worin B, $R_1$ und $R_2$ wie oben festgelegt sind, oder

b) durch in an sich bekannter Weise erfolgendes Umsetzen des 2-(Z)-[Benzal]-cycloheptan-1-[(E)-oxim]-es (Formel I mit A = Hydroxyiminogruppe)

α) mit Halogenalkylaminen der allgemeinen Formel:

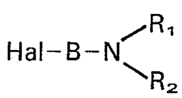 (V)

worin:

Hal für ein Halogenatom, insbesondere Chloratom steht, und

B, $R_1$ und $R_2$ wie oben festgelegt sind, oder

β) durch Umsetzen des ersteren mit einem Dihalogenalkan der allgemeinen Formel:

$$Hal-B-Hal' \qquad (VI)$$

worin:

Hal und Hal' für gleiche oder verschiedene Halogenatome, insbesondere Chloratome, stehen, und

B wie oben festgelegt ist,

und Aminieren der erhaltenen Halogenalkyläther.

6. Verfahren zur Herstellung der 2-(Z)-[Benzal]-1-(E)-[aminoalkoxyimino]-cycloheptane der allgemeinen Formel III, dadurch gekennzeichnet, dass man 2-(E)-[Benzal]-cycloheptan-1-on der Formel:

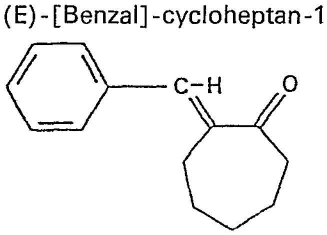 (II)

in einem inerten Lösungsmittel einem Photoisomerisieren durch Bestrahlen mit Licht mit einer Wellenlänge von 200 bis 600 nm unterwirft und danach das erhaltene gegebenenfalls gewonnene, 2-(Z)-[Benzal]-cycloheptan-1-on (Formel I mit A = Sauerstoffatom)

a) in an sich bekannter Weise mit O-(Aminoalkyl)-hydroxylaminderivaten der allgemeinen Formel:

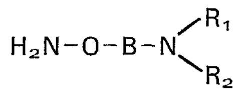 (IV)

worin B, $R_1$ und $R_2$ wie oben festgelegt sind, umsetzt oder

b) in das entsprechende Oxim 2-(Z)-[Benzal]-cycloheptan-1-[(E)-oxim] (Formel I mit A = Hydroxyiminogruppe) überführt und dieses

α) mit Halogenalkylaminen der allgemeinen Formel:

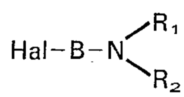 (V)

worin:

Hal für ein Halogenatom, insbesondere Chloratom, steht, und

B, $R_1$ und $R_2$ wie oben festgelegt sind, umsetzt oder

β) mit Dihalogenalkanen der allgemeinen Formel:

$$Hal-B-Hal' \qquad (VI)$$

worin:

Hal und Hal' für gleiche oder verschiedene Halogenatome, insbesondere Chloratome, stehen und

B wie oben festgelegt ist,

umsetzt und die erhaltenen Halogenalkyläther aminiert.

7. Arzneimittel, gekennzeichnet durch einen Gehalt an einer oder beiden der Verbindungen nach Anspruch 1 als Wirkstoff(en), gegebenenfalls zusammen mit einem oder mehr üblichen Konfektionierungsmittel(n).

**Patentansprüche** für den Vertragsstaat: AT

1. Verfahren zur Herstellung von 2-(Z)-[Benzal]-cycloheptan-1-derivaten der allgemeinen Formel:

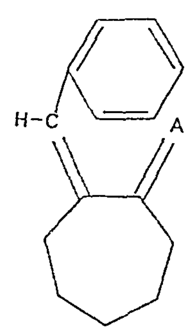 (I)

worin:

A für ein Sauerstoffatom oder eine Hydroxyiminogruppe steht,

dadurch gekennzeichnet, dass man 2-(E)-[Benzal]-cycloheptan-1-on der Formel:

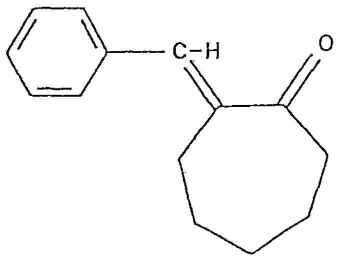

(II)

in einem inerten Lösungsmittel einem Photoisomerisieren durch Bestrahlen mit Licht mit einer Wellenlänge von 200 bis 600 nm unterwirft und danach das erhaltene 2-(Z)-[Benzal]-cycloheptan-1-on (Formel I mit A = Sauerstoffatom) gewinnt und/oder in an sich bekannter Weise in das entsprechende Oxim 2-(Z)-[Benzal]-cycloheptan-1-[(E)-oxim] (Formel I mit A = Hydroxyiminogruppe) überführt.

2. Verfahren nach Anspruch 1, dadurch gekennzeichnet, dass man das Bestrahlen mit Licht mit einer Wellenlänge von 300 bis 600 nm durchführt.

3. Verfahren nach Anspruch 2, dadurch gekennzeichnet, dass man als inertes Lösungsmittel einen aliphatischen Alkohol, insbesondere Methanol, verwendet.

4. Verwendung der 2-(Z)-[Benzal]-cycloheptan-1-derivate, erhalten nach Anspruch 1, zur Herstellung von 2-(Z)-[Benzal]-1-(E)-[aminoalkoxyimino]-cycloheptanen der allgemeinen Formel:

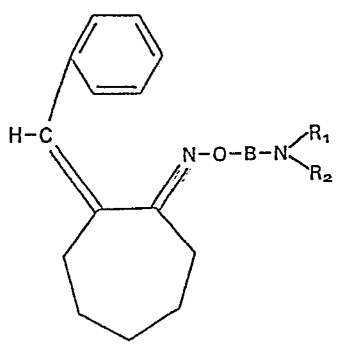

(III)

worin:

B für einen geradkettigen oder verzweigten Alkylenrest mit 2 bis 4 Kohlenstoffatomen steht, und

$R_1$ und $R_2$ Wasserstoff oder einen Alkylrest mit 1 bis 4 Kohlestoffatom(en) bedeuten, oder

$R_1$ und $R_2$ zusammen eine, gegebenenfalls durch ein Sauerstoffatom oder ein, gegebenenfalls durch einen Alkylrest mit 1 bis 3 Kohlenstoffatom(en) oder einen Benzylrest substituiertes, Stickstoffatom unterbrochene, Alkylenkette mit 4 bis 7 Kohlenstoffatomen darstellen,

a) durch in an sich bekannter Weise erfolgendes Umsetzen des 2-(Z)-[Benzal]-cycloheptan-1-ones (Formel I mit A = Sauerstoffatom) mit O-(Aminoalkyl)-hydroxylaminderivaten der allgemeinen Formel:

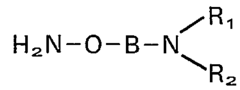

(IV)

worin B, $R_1$ und $R_2$ wie oben festgelegt sind, oder

b) durch in an sich bekannter Weise erfolgendes Umsetzen des 2-(Z)-[Benzal]-cycloheptan-1-[(E)-oxim]-es (Formel I mit A = Hydroxyiminogruppe),

α) mit Halogenalkylaminen der allgemeinen Formel:

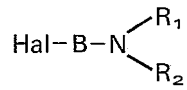

(V)

worin:

Hal für ein Halogenatom, insbesondere Chloratom steht, und

B, $R_1$ udn $R_2$ wie oben festgelegt sind, oder

β) durch Umsetzen des ersteren mit einem Dihalogenalkan der allgemeinen Formel:

Hal−B−Hal′ (VI)

worin:

Hal und Hal′ für gleiche oder verschiedene Halogenatome, insbesondere Chloratome, stehen, und

B wie oben festgelegt ist,

und Aminieren der erhaltenen Halogenalkyläther.

5. Verfahren zur Herstellung der 2-(Z)-[Benzal]-1-(E)-[aminoalkoxyimino]-cycloheptane der allgemeinen Formel III, dadurch gekennzeichnet, dass man 2-(E)-[Benzal]-cycloheptan-1-on der Formel:

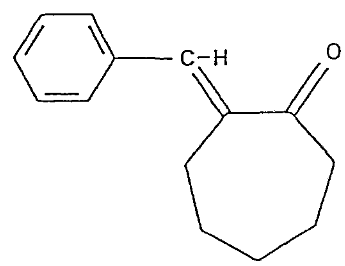

(II)

in einem inerten Lösungsmittel einem Photoisomerisieren durch Bestrahlen mit Licht mit einer Wellenlänge von 200 bis 600 nm unterwirft und danach das erhaltene gegebenenfalls gewonnene, 2-(Z)-[Benzal]-cycloheptan-1-on (Formel I mit A = Sauerstoffatom)

a) in an sich bekannter Weise mit O-(Aminoalkyl)-hydroxylaminderivaten der allgemeinen Formel:

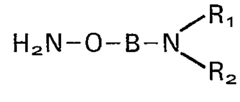

(IV)

worin B, $R_1$ und $R_2$ wie oben festgelegt sind, umsetzt oder

b) in das entsprechende Oxim 2-(Z)-[Benzal]-cycloheptan-1-[(E)-oxim] (Formel I mit A = Hydroxyiminogruppe) überführt und dieses

α) mit Halogenalkylaminen der allgemeinen Formel:

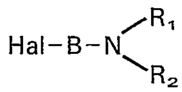

(V)

worin:

Hal für ein Halogenatom, insbesondere Chloratom, steht und

B, $R_1$ und $R_2$ wie oben festgelegt sind, umsetzt oder

β) mit Dihalogenalkanen der allgemeinen Formel:

Hal–B–Hal' (VI)

worin:

Hal und Hal' für gleiche oder verschiedene Halogenatome, insbesondere Chloratome, stehen und

B wie oben festgelegt ist, umsetzt und die erhaltenen Halogenalkyläther aminiert.

**Claims** for the Contracting States BE, CH, DE, FR, GB, IT, LI, NL, SE

1. 2-(Z)-[Benzal]-cycloheptane-1 derivatives having the general formula:

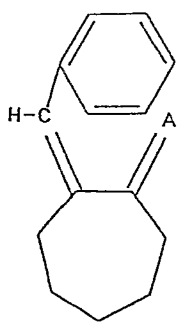

(I)

wherein:

A represents an oxygen atom or a hydroxyimino group.

2. A process for preparing the 2-(Z)-[benzal]-cycloheptane-1 derivatives according to Claim 1, characterized in that one subjects 2-(E)-[benzal]-cycloheptane-1-one having the formula:

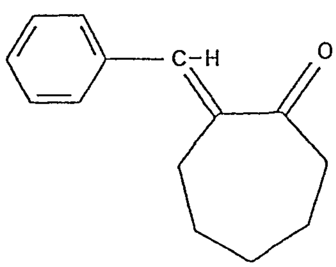

(II)

in an inert solvent to a photoisomerisation by irradiation with light of a wave length of 200 to 600 nm and thereafter one gains the obtained 2-(Z)-[benzal]-cycloheptane-1-one (formula I with A = oxygen atom) and/or one converts it in a manner known per se into the corresponding oxime 2-(Z)-[benzal]-cycloheptane-1-[(E)-oxime] (formula I with A = hydroxyimino group).

3. A process according to Claim 2, characterized in that one carries out the irradiation with light having a wave length of from 300 to 600 nm.

4. A process according to Claim 3, characterized in that one uses as an inert solvent an aliphatic alcohol, particularly methanol.

5. The use of the 2-(Z)-[benzal]-cycloheptane-1 derivatives according to Claim 1 for the preparation of 2-(Z)-[benzal]-1-(E)-[aminoalkoxyimino]-cycloheptanes having the general formula:

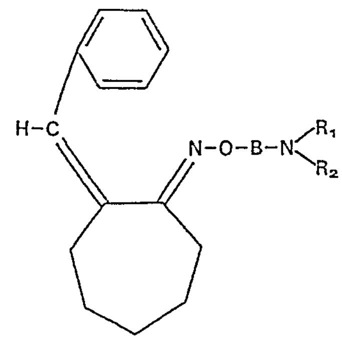

(III)

wherein:

B represents a straight-chain or branched-chain alkylene radical haing from 2 to 4 carbon atoms, and

$R_1$ and $R_2$ mean hydrogen or an alkyl radical having from 1 to 4 carbon atom(s), or

$R_1$ and $R_2$ together represent an alkylene chain having from 4 to 7 carbon atoms optionally interrupted by an oxygen atom or a nitrogen atom optionally substituted by an alkyl radical having from 1 to 3 carbon atom(s) or a benzyl radical,

a) by reacting in a manner known per se the 2-(Z)-[benzal]-cycloheptane-1-one (formula I with A = oxygen atom) with O-(aminoalkyl)-hydroxylamine derivatives having the general formula:

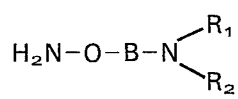

(IV)

wherein B, $R_1$ and $R_2$ are as above defined, or

b) by reacting in a manner known per se the 2-(Z)-[benzal]-cycloheptane-1-[(E)-oxime] (formula I with A = hydroxyimino group)

α) with halogenalkylamiens having the general formula:

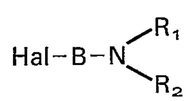

(V)

wherein:

Hal represents a halogen atom, particularly chlorine atom, and

B, $R_1$ and $R_2$ are as defined above, or

β) by reacting the former with a dihalogenalkane having the general formula:

Hal–B–Hal' (VI)

wherein:

Hal and Hal' represent identical or different halogen atoms, particularly chlorine atoms, and

B is as defined above,

and aminating the obtained halogenalkylether.

6. A process for preparing the 2-(Z)-[benzal]-1-(E)-[aminoalkoxyimino]-cycloheptanes having the general formula III,characterized in that

one subjects 2-(E)-[benzal]-cycloheptane-1-one having the formula:

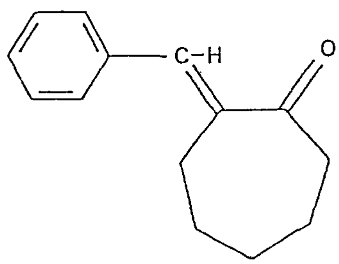

(II)

in an inert solvent to a photoisomeriation by irradiation with light having a wave length of from 200 to 600 nm and thereafter

a) one reacts the obtained 2-(Z)-[benzal]-cycloheptane-1-one (formula I with A = oxygen atom) optionally gained in a manner known per se with O-(aminoalkyl)-hydroxylamine derivatives having the general formula:

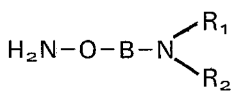

(IV)

wherein B, $R_1$ and $R_2$ are as defined above, or

b) one converts the 2-(Z)-[benzal]-cycloheptane-1-one (formula I with A = oxygen atom) optionally gained into the corresponding oxime 2-(Z)-[benzal]-cycloheptane-1-[(E)-oxime] (formula I with A = hydroxyimino group) and

α) one reacts this with halogenalkylamines having the general formula:

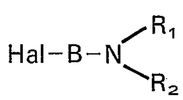

(V)

wherein:

Hal represents a halogen atom, particularly chlorine atom, and

B, $R_1$ and $R_2$ are as defined above, or

β) one reacts this {oxime 2-(Z)-[benzal]-cycloheptane-1-[(E)-oxime]} with dihalogenalkanes having the general formula:

Hal–B–Hal′ (VI)

wherein:

Hal and Hal′ represent identical or different halogen atoms, particularly chlorine atoms, and

B is as defined above and one aminates the obtained halogenalkylethers.

7. Medicaments, characterized by a content of 1 or both of the compounds according to Claim 1 as [an] active principle(s), optionally together with 1 or more usual processing agent(s).

**Claims** for the Contracting State: AT

1. A process for preparing 2-(Z)-[Benzal]-cycloheptane-1 derivatives having the general formula:

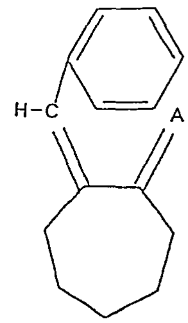

(I)

wherein:

A represents an oxygen atom or a hydroxyimino group,

characterized in that one subjects 2-(E)-[benzal]-cycloheptane-1-one having the formula:

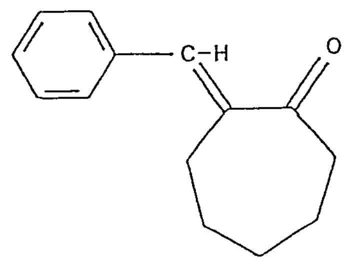

(II)

in an inert solvent to a photoisomerisation by irradiation with light of a wave length of 200 to 600 nm and thereafter one gains the obtained 2-(Z)-[benzal]-cycloheptane-1-one (formula I with A = oxygen atom) and/or one converts it in a manner known per se into the corresponding oxime 2-(Z)-[benzal]-cycloheptane-1-[(E)-oxime] (formula I with A = hydroxyimino group).

2. A process according to Claim 1, characterized in that one carries out the irradiation with light having a wave length of from 300 to 600 nm.

3. A process according to Claim 2, characterized in that one uses as an inert solvent an aliphatic alcohol, particularly methanol.

4. The use of the 2-(Z)-[benzal]-cycloheptane-1 derivatives prepared according to Claim 1 for the preparation of 2-(Z)-[benzal]-1-(E)-[aminoalkoxyimino]-cycloheptanes having the general formula:

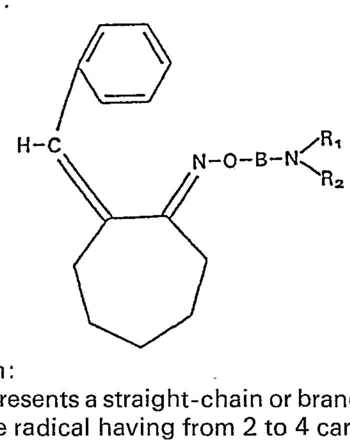

(III)

wherein:

B represents a straight-chain or branched-chain alkylene radical having from 2 to 4 carbon atoms, and

$R_1$ and $R_2$ mean hydrogen or an alkyl radical having from 1 to 4 carbon atom(s), or

$R_1$ and $R_2$ together represent an alkylene chain having from 4 to 7 carbon atoms optionally interrupted by an oxygen atom or a nitrogen atom optionaly substituted by an alkyl radical having from 1 to 3 carbon atom(s) or a benzyl radical,

a) by reacting in a manner known per se the 2-(Z)-[benzal]-cycloheptane-1-one (formula I with A = oxygen atom) with O-(aminoalkyl)-hydroxylamine derivatives having the general formula:

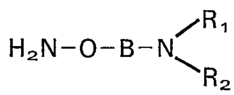

(IV)

wherein B, $R_1$ and $R_2$ are as above defined, or

b) by reacting in a manner known per se the 2-(Z)-[benzal]-cycloheptane-1-[(E)-oxime] (formula I with A = hydroxyimino group)

α) with halogenalkylamines having the general formula:

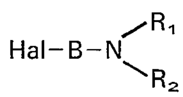

(V)

wherein:

Hal represents a halogen atom, particularly chlorine atom, and

B, $R_1$ and $R_2$ are as defined above, or

β) by reacting the former with a dihalogenalkane having the general formula:

Hal−B−Hal' (VI)

wherein:

Hal and Hal' represent identical or different halogen atoms, particularly chlorine atoms, and

B is as defined above,

and aminating the obtained halogenalkylether.

5. A process for preparing the 2-(Z)-[benzal]-1-(E)-[aminoalkoxyimino]-cycloheptanes having the general formula III, characterized in that one subjects 2-(E)-[benzal]-cycloheptane-1-one having the formula:

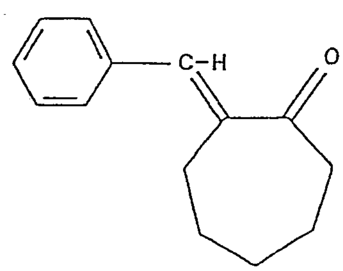

(II)

in an inert solvent to a photoisomerisation by irradiation with light having a wave length of from 200 to 600 nm and thereafter

a) one reacts the obtained 2-(Z)-[benzal]-cycloheptane-1-one (formula I with A = oxygen atom) optionally gained in a manner known per se with O-(aminoalkyl)-hydroxylamine derivatives having the general formula:

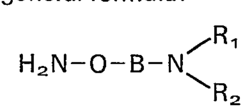

(IV)

wherein B, $R_1$ and $R_2$ are as defined above, or

b) one converts the 2-(Z)-[benzal]-cycloheptane-1-one (formula I with A = oxygen atom) optionally gained into the corresponding oxime 2-(Z)-[benzyl]-cycloheptane-1-[(E)-oxime] (formula I with A = hydroxyimino group) and

α) one reacts this with halogenalkylamines having the general formula:

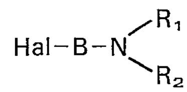

(V)

wherein:

Hal represents a halogen atom, particularly chlorine atom, and

B, $R_1$ and $R_2$ are as defined above, or

β) one reacts this {oxime 2-(Z)-[benzal]-cycloheptane-1-[(E)-oxime]} with dihalogenalkanes having the general formula:

Hal−B−Hal' (VI)

wherein:

Hal and Hal' represent identical or different halogen atoms, particularly chlorine atoms, and

B is as defined above and

one aminates the obtained halogenalkylethers.

**Revendications** pour les Etats contractants BE, CH, DE, FR, GB, IT, LI, NL, SE

1. Dérivés de 2-(Z)-[benzal]-cycloheptane-1 de formule générale:

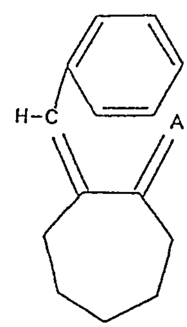

(I)

dans laquelle A est un atome d'oxygène ou un groupe hydroxyimino.

2. Procédé pour la préparation des dérivés de 2-(Z)-[benzal]-cycloheptane-1 suivant la revendication 1, caractérisé en ce qu'on soumet la 2-(E)-[benzal]-cycloheptane-1-one de formule:

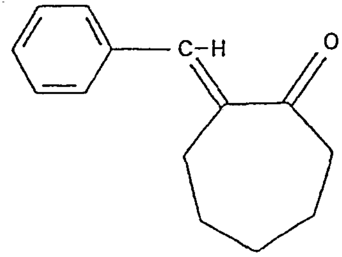

(II)

dans un solvant inerte, à une photoisomérisation par irradiation avec de la lumière ayant une longueur d'onde de 200 à 600 nm, puis on récupère la 2-(Z)-[benzal]-cycloheptane-1-one obtenue (formule I dans laquelle A = oxygène) et/ou on la

convertit d'une manière en soi connue en oxime correspondante 2-(Z)-[benzal]-cycloheptane-1-[(E)-oxime] (formule I dans laquelle A = un groupe hydroxyimino).

3. Procédé suivant la revendication 2, caractérisé en ce qu'on effectue l'irradiation avec de la lumière ayant une longueur d'onde de 300 à 600 nm.

4. Procédé suivant la revendication 3, caractérisé en ce qu'on utilise comme solvant inerte un alcool aliphatique, en particulier du méthanol.

5. Utilisation des dérivés de 2-(Z)-[benzal]-cycloheptane-1 suivant la revendication 1, pour la préparation de 2-(Z)-[benzal]-1-(E)-aminoalcoxyimino)-cycloheptanes de formule générale:

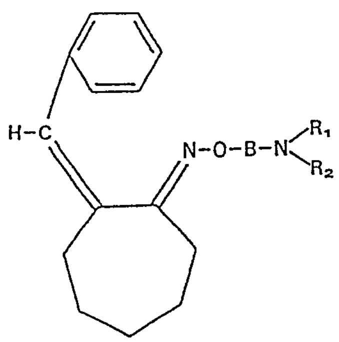

(III)

dans laquelle:

B est un radical alcoylène linéaire ou ramifié ayant de 2 à 4 atomes de carbone, et

$R_1$ et $R_2$ désignent de l'hydrogène ou un radical alcoyle ayant de 1 à 4 atomes de carbone, ou

$R_1$ et $R_2$ représentent ensemble une chaîne alcoylène ayant de 4 à 7 atomes de carbone, éventuellement interrompue par un atome d'oxygène ou un atome d'azote éventuellement substitué par un radical alcoyle ayant de 1 à 3 atomes de carbone ou par un radical benzyle,

a) par une réaction s'effectuant d'une manière en soi connue, de la 2-(Z)-[benzal]-cycloheptan-1-one (formule I dans laquelle A = oxygène) avec des dérivés d'O-(aminoalcoyl)-hydroxylamine de formule générale:

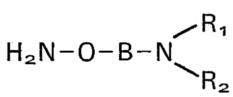

(IV)

dans laquelle B, $R_1$ et $R_2$ ont la signification indiquée précédemment, ou

b) par une réaction s'effectuant d'une manière en soi connue de la 2-(Z)-[benzal]-cycloheptane-1-[(E)-oxime] (formule I dans laquelle A = groupe hydroxyimino).

α) avec des halogénoalcoylamines de formule générale:

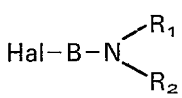

(V)

dans laquelle:

Hal désigne un atome d'halogène, en particulier un atome de chore, et

B, $R_1$ et $R_2$ ont la signification indiquée précédemment, ou

β) par réaction de la première avec un dihalogénoalcane de formule générale:

Hal–B–Hal' (VI)

dans laquelle:

Hal et Hal' désignent des atomes d'halogène identiques ou différents, en particulier des atomes de chlore, et B a la signification indiquée précédemment, et animation des éthers halogénoalcoyliques obtenus.

6. Procédé de préparation des 2-(Z)-[benzal]-1-(E)-[aminoalcoxyimino]-cycloheptanes de formule générale III, caractérisé en ce qu'on soumet la 2-(E)-[benzal]-cycloheptan-1-one de formule:

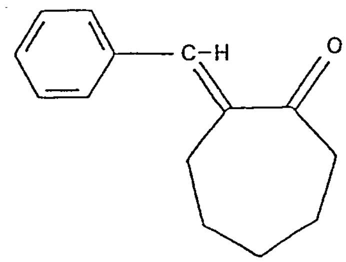

(II)

dans un solvant inerte, à une photoisomérisation par irradiation avec de la lumière ayant une longueur d'ondes de 200 à 600 nm, puis à partir de la 2-(Z)-[benzal]-cycloheptan-1-one obtenue, éventuellement récupérée (formule I dans laquelle A = oxygène),

a) on la fait réagir d'une manière en soi connue avec des dérivés d'O-(aminoalcoyl)hydroxylamine de formule générale:

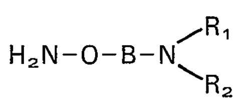

(IV)

dans laquelle B, $R_1$ et $R_2$ ont la signification indiquée précédemmemnt, ou

b) on la convertit en oxime correspondante 2-(Z)-[benzal]-cycloheptane-1-[(E)-oxime] (formule I dans laquelle A = groupe hydroxyimino), et

α) on fait réagir celle-ci avec des halogénoalcoylamines de formule générale:

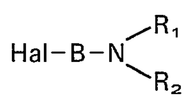

(V)

dans laquelle:

Hal est un atome d'halogène, en particulier un atome de chlore, et

B, $R_1$ et $R_2$ ont la signification indiquée précédemment, ou bien

β) on la fait réagir avec des dihalogénoalcanes de formule générale:

Hal–B–Hal' (VI)

dans laquelle:

Hal et Hal' désigent des atomes d'halogène identiques ou différents, en particulier des atomes de chlore, et

B a la signification indiquée précédement, et on effectue l'amination des éthers halogénoalcoyliques obtenus.

7. Médicaments, caractérisés en ce qu'ils contiennent l'un ou les deux composés suivant la revendication 1 à titre de substance(s) active(s), éventuellement conjointement à un ou plusieurs excipients usuels.

**Revendications** pour Etat contractant: AT

1. Procédé pour la préparation de dérivés de 2-(Z)-[benzal]-cycloheptane-1 de formule générale:

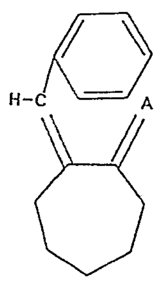

(I)

dans laquelle A est un atome d'oxygène ou un groupe hydroxyimino, caractérisé en ce qu'on soumet la 2-(E)-[benzal]-cycloheptan-1-one de formule:

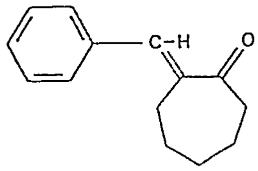

(II)

dans un solvant inerte, à une photoisomérisation par irradiation avec de la lumière ayant une longueur d'onde de 200 à 600 nm, puis on récupère la 2-(Z)-[benzal]-cycloheptan-1-one obtenue (formule I dans laquelle A = oxygène) et/ou on la convertit d'une manière en soi connue en oxime correspondante 2-(Z)-[benzal]-cycloheptane-1-[(E)-oxime] (formule I dans laquelle A = un groupe hydroxyimino).

2. Procédé suivant la revendication 1, caractérisé en ce qu'on effectue l'irradiation avec de la lumière ayant une longueur d'onde de 300 à 600 nm.

3. Procédé suivant la revendication 2, caractérisé en ce qu'on utilise comme solvant inerte un alcool aliphatique, en particulier du méthanol.

4. Utilisation des dérivés de 2-(Z)-[benzal]-cycloheptane-1 préparés suivant la revendication 1 pour la préparation de 2-(Z)-[benzal]-1-(E)-(aminoalcoxyimino)-cycloheptanes de formule générale:

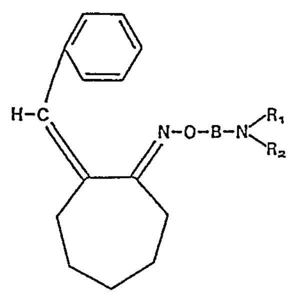

(III)

dans laquelle:

B est un radical alcoylène linéaire ou ramifié ayant de 2 à 4 atomes de carbone, et

$R_1$ et $R_2$ désignant de l'hydrogène ou un radical alcoyle ayant de 1 à 4 atomes de carbone, ou

$R_1$ et $R_2$ représentent ensemble une chaîne alcoylène ayant de 4 à 7 atomes de carbone, éventuellement interrompue par un atome d'oxygène ou un atome d'azote éventuellement substitué par un radical alcoyle ayant de 1 à 3 atomes de carbone ou par un radical benzyle,

a) par une réaction s'effectuant d'une manière en soi connue, de la 2-(Z)-[benzal]-cycloheptan-1-one (formule I dans laquelle A = oxygène) avec des dérivés d'O-(aminoalcoyle)-hydroxylamine de formule générale:

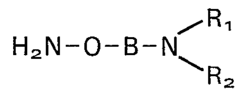

(IV)

dans laquelle B, $R_1$ et $R_2$ ont la signification indiquée précédemment, ou

b) par une réaction s'effectuant d'une manière en soi connue de la 2-(Z)-[benzal]-cycloheptane-1[(E)-oxime] (formule I dans laquelle A = groupe hydroxyimino),

α) avec des halogénoalcoylamines de formule générale:

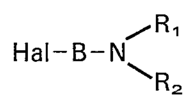

(V)

dans laquelle:

Hal désigne un atome d'halogène, en particulier un atome de chlore, et

B, $R_1$ et $R_2$ ont la signification indiquée précédemment, ou

β) par réaction de la première avec un dihalogénoalcane de formule générale:

Hal−B−Hal' (VI)

dans laquelle:

Hal et Hal' désignent des atomes d'halogène identiques ou différents, en particulier des atomes de chlore, et

B a la signification indiquée précédemment, et amination des éthers halogénoalcoyliques obtenus.

5. Procédé de préparation des 2-(Z)-[benzal]-1-(E)-[aminoalcoxyimino]-cycloheptanes de formule générale III, caractérisé en ce qu'on soumet la 2-(E)-[benzal]-cycloheptane-1-one de formule:

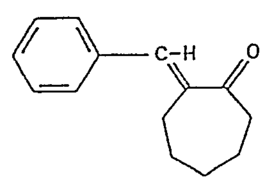

(II)

dans un solvant inerte, à une photoisomérisation par irradiation avec de la lumière ayant une longueur d'onde de 200 à 600 nm, puis à partir de la 2-(Z)-[benzal]-cycloheptan-1-one obtenue, éventuellement récupérée (formule I dans laquelle A = oxygène),

a) on fait réagir d'une manière en soi connue avec des dérivés d'O-(aminoalcoyl)hydroxylamine de formule générale:

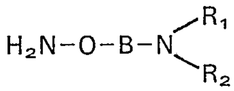
(IV)

dans laquelle B, $R_1$ et $R_2$ ont la signification indiquée précédemment, ou

b) on la convertit en oxime correspondante 2-(Z)-[benzal]-cycloheptane-1-[(E)-oxime] (formule I dans laquelle A = groupe hydroxyimino), et

α) on fait réagir celle-ci avec des halogénoalcoylamines de formule générale:

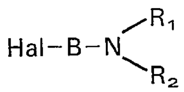
(V)

dans laquelle:

Hal est un atome d'halogène, en particulier un atome de chlore, et

B, $R_1$ et $R_2$ ont la signification indiquée précédemment, ou bien

β) on la fait réagir avec des dihalogénoalcanes de formule générale:

$$Hal-B-Hal' \qquad (VI)$$

dans laquelle:

Hal et Hal' désignent des atomes d'halogène identiques ou différents, en particulier des atomes de chlore, et

B a la signification indiquée précédemment, et on effectue l'amination des éthers halogénoalcoyliques obtenus.